# EUROPEAN PATENT APPLICATION

(11) **EP 2 417 897 A1**
(43) Date of publication of application: **15.02.2012**
(21) Application number: 10761602.1
(22) Date of filing: 26.03.2010
(51) Int. Cl.: A61B 1/00, A61B 1/06

(54) **ENDOSCOPIC DEVICE**

(30) Priority: 09.04.2009 JP 2009095040
(71) Applicant: OLYMPUS MEDICAL SYSTEMS CORP., Tokyo (JP)
(72) Inventor: GONO, Kazuhiro, Tokyo 151-0072 (JP)
(74) Representative: Schicker, Silvia
(86) International application number: PCT/JP2010/055415
(87) International publication number: WO 2010/116902

(57) **Abstract**

An endoscope apparatus of the invention includes: an endoscope including an objective optical system capable of changing an observation magnification; a light source device capable of selectively emitting broadband light including a visible light region and narrow-band lights of a plurality of wavelength bands obtained by discretizing light in the visible light region; an observation magnification changing instruction section for issuing a magnification changing instruction for gradually increasing or decreasing the observation magnification of the objective optical system; and a mode switching section for switching the light emitted from the light source device from one light to another light when detecting that the observation magnification gradually changes to reach a predetermined observation magnification based on the magnification changing instruction.

## Description

### Technical Field

The present invention relates to an endoscope apparatus and more particularly to an endoscope apparatus in which observation magnification can be changed.

### Background Art

Conventionally, endoscope apparatuses configured by including an endoscope, a light source device and the like have been widely used in a medical field and the like. In particular, the endoscope apparatuses in the medical field are mainly used for observation in a living body by a surgeon and the like.

In addition, generally known observation using such endoscope apparatuses in the medical field includes a normal light observation in which an image having a color substantially the same as a color under bare eye observation can be obtained by irradiating an object in a living body with light including R(red), G(green) and B(Blue) colors, for example, and a narrow-band light observation in which an image containing enhanced images of blood vessels and the like existing on a mucosa surface layer in a living body can be obtained by irradiating the object with light of a narrower wavelength band than that of the illumination light in the normal light observation. Japanese Patent Application Laid-Open Publication No. 2007-020728 discloses an endoscope apparatus configured to be capable of switching between modes corresponding to the above-described two kinds of observations.

In some endoscope apparatuses in the medical field, it is possible to observe a local region of an object existing in a living body with several tens to several hundreds of observation magnification.

When performing endoscope observation of a local region of an object existing in a living body, the user typically performs an operation to change the observation magnification from low magnification side to high magnification side while moving a distal end portion of the endoscope closer to a desired region. When the user performs the aforementioned operation while viewing an image obtained in normal light observation, combination of shallow depth of field of an optical system provided to the endoscope and low contrast of finding that serves as a clue for focus adjustment (at least one of mucosa surface microstructure and capillary vessel pattern) causes a problem of difficulty in focusing on the observation target.

In narrow-band light observation in contrast, an image can be obtained with improved contrast of finding that serves as a clue for focus adjustment (at least one of mucosa surface microstructure and capillary vessel pattern) when performing the aforementioned operation. Nevertheless, in order to obtain such an image when performing narrow-band light observation using an endoscope, it is necessary to decrease to some extent the distance between the observation target and a distal end portion of the endoscope.

On the other hand, Japanese Patent Application Laid-Open Publication No. 2007-020728 discloses a technique to switch between normal light observation mode and narrow-band light observation mode at a border of a predetermined observation magnification, but fails to refer to switching between the both observation modes assuming the aforementioned operation, that is, the operation that the user actually performs during endoscope observation of a local region of an object existing in a living body.

For this reason, the technique recited in Japanese Patent Application Laid-Open Publication No. 2007-020728 causes a problem of easy occurrence of a situation where no appropriate narrow-band light observation image is obtained when performing endoscope observation of a local region of an object existing in a living body, which results in a longer time period required for focus adjustment.

The present invention was made in view of the aforementioned circumstance and has an objective to provide an endoscope apparatus capable of shortening the time period required for focus adjustment when performing endoscope observation of a local region of an object existing in a living body, compared to conventional arts.

### Disclosure of Invention

### Means for Solving the Problem

An endoscope apparatus according to the present invention includes: an endoscope including an objective optical system capable of changing an observation magnification; a light source device capable of selectively emitting broadband light including a visible light region and narrow-band lights of a plurality of wavelength bands obtained by discretizing light in the visible light region; an observation magnification changing instruction section for issuing a magnification changing instruction for gradually increasing or decreasing the observation magnification of the objective optical system; and a mode switching section for switching the light emitted from the light source device from one light to another light when detecting that the observation magnification gradually changes to reach a predetermined observation magnification based on the magnification changing instruction.

### Brief Description of the Drawings

FIG 1 is a view showing a main part of an endoscope apparatus according to an embodiment of the present invention.
FIG 2 is a view showing one example of a configuration of an objective optical system of an endoscope in FIG. 1.
FIG. 3 is a view showing one example of a configuration of a rotary filter of a light source device in FIG 1.
FIG. 4 is a view showing one example of transmission characteristics of filters in a first filter group in FIG. 3
FIG. 5 is a view showing one example of transmission characteristics of filters in a second filter group in FIG. 3.

### Best Mode for Carrying Out the Invention

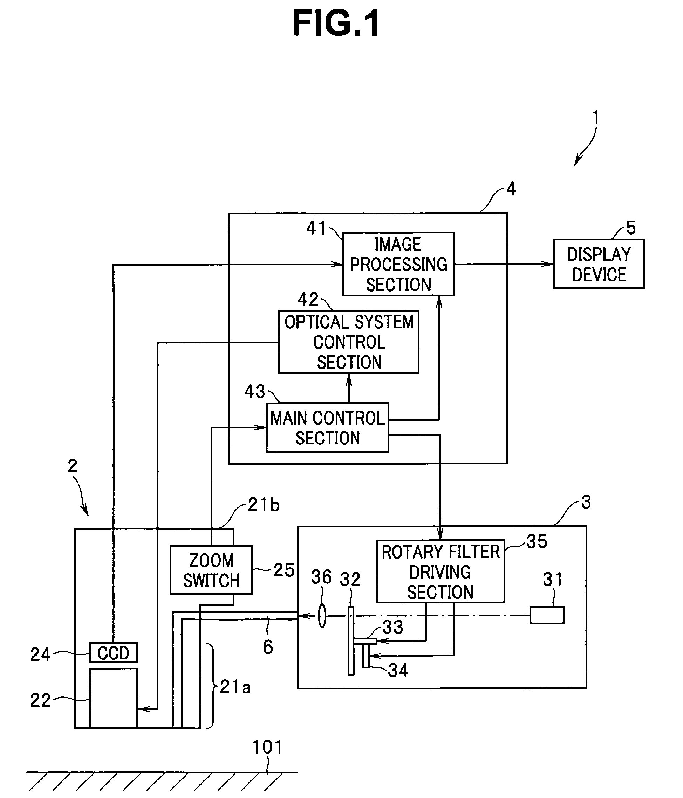

Hereinafter, an embodiment of the present invention will be described with reference to drawings. FIGS. 1 to 5 relate to the embodiment of the present invention.

As shown in FIG. 1, an endoscope apparatus 1 includes: an endoscope 2 which is configured to be capable of changing an observation magnification and to output a picked-up image of an object 101 as an image pickup signal; a light source device 3 which emits illumination light for illuminating the object 101 in a body cavity of a test subject; a processor 4 which generates a video signal by performing image processing on the image pickup signal from the endoscope 2 and outputs the generated video signal; and a display device 5 which displays an image corresponding to the video signal from the processor 4.

A light guide 6 for transmitting the illumination light emitted from the light source device 3 to a distal end portion 21a of the endoscope 2 is inserted through the endoscope 2.

One end face (incident end face) of the light guide 6 is connected to the light source device 3. In addition, the other end face (emission end face) of the light guide 6 is arranged in the vicinity of an illumination optical system, not shown, provided at the distal end portion 21a of the endoscope 2. According to such a configuration, the illumination light emitted from the light source device 3 passes through the light guide 6 and the illumination optical system, not shown, to be emitted to the object 101.

At the distal end portion 21a of the endoscope 2, an objective optical system 22 which forms an image of an object and a CCD 24 which picks up the image of the object formed through the objective optical system 22. In addition, a zoom switch 25 for issuing a magnification changing instruction is provided at an operation portion 21 b located on a proximal end side (rear end side) of the endoscope 2.

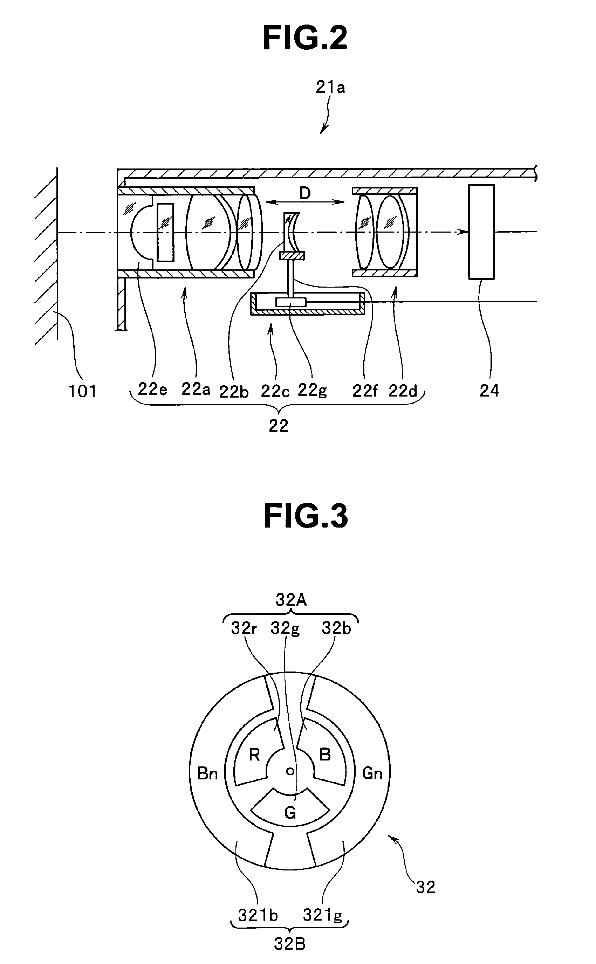

As shown in FIG. 2, the objective optical system 22 having a magnification changing function includes: a first lens group 22a provided on the distal-most side of the distal end portion 21a; a mobile optical system 22b which is capable of being displaced along the optical axis thereof and which receives at a front face thereof the light passed through the first lens group 22a; a focus position adjusting section 22c capable of moving the mobile optical system 22b in a direction along the optical axis (direction along the arrow D in FIG. 2); and a second lens group 22d which receives at a front face thereof the light passed through the mobile optical system 22b.

The first lens group 22a includes a plurality of lenses the positions of which are fixed. The plurality of lenses includes at least a distal end lens 22e on which the light from the object 101 is incident.

The focus position adjusting section 22c is configured by using a linear actuator, for example. Specifically, the focus position adjusting section 22c includes: an arm 22f connected to a side portion of the mobile optical system 22b and an arm driving section 22g which moves the arm 22f in the direction along the arrow D in FIG. 2 according to the control by the processor 4. According to such a configuration, the mobile optical system 22b moves in conjunction with the operation of the focus position adjusting section 22c, which changes the focus position and the observation magnification of the objective optical system 22.

The second lens group 22d includes a plurality of lenses the positions of which are fixed and forms an image of the light incident through the first lens group 22a and the mobile optical system 22b on an image pickup surface of the CCD 24.

The zoom switch 25 having a function as an observation magnification changing instruction section includes a button, a lever, and the like for continuously outputting the magnification changing instruction to the processor 4 in the period during which operation (of depression or the like) is performed by a user. According to such a configuration of the zoom switch 25, the observation magnification of the objective optical system 22 is gradually increased or decreased during the operation of the zoom switch 25 by the user.

The light source device 3 includes: a white light source 31 configured of a xenon lamp and the like; a rotary filter 32 which converts the white light emitted from the white light source 31 into frame-sequential illumination light; a motor 33 which rotates and drives the rotary filter 32; a motor 34 which moves the rotary filter 32 and the motor 33 in a direction perpendicular to an emission optical path of the white light source 31; a rotary filter driving section 35 which drives the motors 33, 34 based on the control by the processor 4; and a light condensing optical system 36 which condenses the illumination light passed through the rotary filter 32 to supply the condensed illumination light to the incident end face of the light guide 6.

As shown in FIG 3, the rotary filter 32 is formed in a shape of a disk having a rotational axis at the center thereof, and includes a first filter group 32A having a plurality of filters arranged along a circumferential direction on an inner peripheral side and a second filter group 32B having a plurality of filters arranged along a circumferential direction on an outer peripheral side. The driving force of the motor 33 is transmitted to the rotational axis, and thereby the rotary filter 32 is rotated. Note that the rotary filter 32 is configured of a light-shielding member except for the portions where the first filter group 32A and the second filter group 32B are arranged.

The first filter group 32A includes an R filter 32r which transmits light of red wavelength band, a G filter 32g which transmits light of green wavelength band, and a B filter 32b which transmits light of blue wavelength band which are arranged along the circumferential direction on the inner peripheral side of the rotary filter 32.

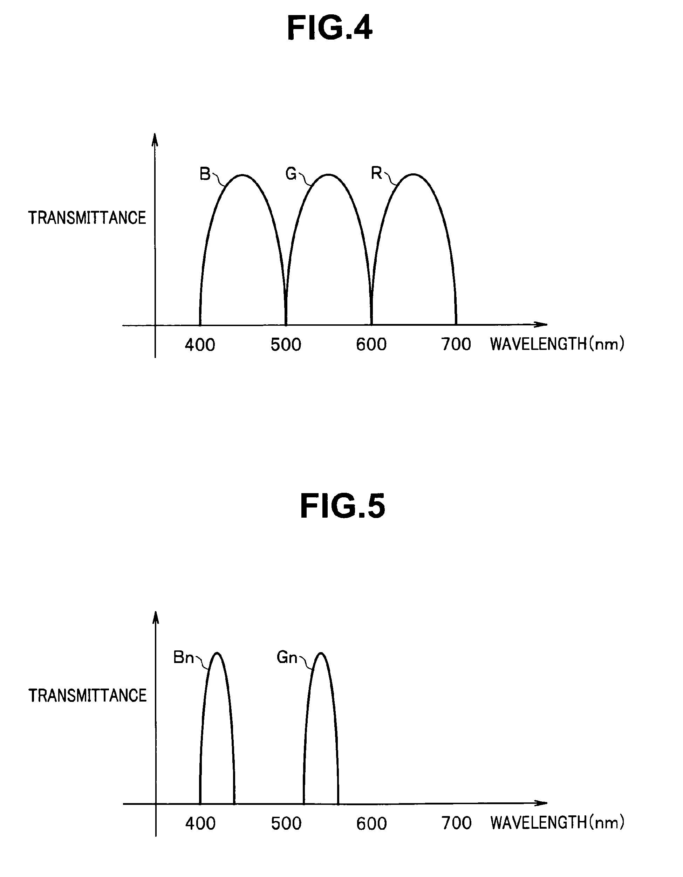

The R filter 32r is configured to transmit light (R light) of wavelengths 600 nm to 700 nm, as shown in FIG. 4, for example. The G filter 32g is configured to transmit light (G light) of wavelengths 500 nm to 600 nm, as shown in FIG. 4, for example. Furthermore, the B filter 32b is configured to transmit light (B light) of wavelengths 400 nm to 500 nm, as shown in FIG 4, for example.

That is, the white light emitted from the white light source 31 passes through the first filter group 32A, thereby being converted into broadband light for normal light observation mode.

The second filter group 32B includes a Bn filter 321b which transmits blue narrow-band light and a Gn filter 321 g which transmits green narrow-band light, which are arranged along the circumferential direction on the outer peripheral side of the rotary filter 32.

The Bn filter 321b is configured to transmit the narrow-band light (Bn light) on the short wavelength side of the B light, as shown in FIG 5, for example.

In addition, the Gn filter 321 g is configured to transmit the narrow-band light (Gn light), the center wavelength of which is around 540 nm, as shown in FIG. 5, for example.

That is, the white light emitted from the white light source 31 is converted into discrete lights through the second filter group 32B to be narrow-band lights of a plurality of wavelength bands for narrow-band light observation mode.

The processor 4 includes an image processing section 41, an optical system control section 42, a main control section 43 which performs control according to the magnification changing instruction by the zoom switch 25.

The image processing section 41 sequentially performs noise removal processing, A/D conversion processing, image generating processing, D/A conversion processing and the like on the inputted image pickup signal based on the control by the main control section 43, to thereby generate a video signal and output the generated video signal to the display device 5.

The optical system control section 42 causes a focus position adjusting section 22c to operate such that a mobile optical system 22b is disposed at a position corresponding to the observation magnification, based on the control by the main control section 43.

The main control section 43 including a function as a mode switching section constantly monitors the magnification changing instruction by the zoom switch 25, and performs control to switch the operations of the rotary filter driving section 35, the image processing section 41, and the optical system control section 42 (control to switch between the normal light observation mode and the narrow-band light observation mode) according to the result of monitoring.

Here, the working of the endoscope apparatus 1 will be described. Note that description will be made assuming that each of the sections in the endoscope apparatus 1 is activated in the normal light observation mode in which the observation magnification is unmagnified in the initial state at the time of power-on.

When the power source of the processor 4 is turned on, the main control section 43 controls the rotary filter driving section 35, the image processing section 41, and the optical system control section 42 to operate in the normal light observation mode in which the observation magnification is unmagnified.

Then, the rotary filter driving section 35 drives the motors 33, 34 based on the control by the main control section 43 such that the first filter group 32A is interposed on the optical path of the white light source 31. In addition, based on the control by the main control section 43, the image processing section 41 performs operation to generate a normal light observation image (full-color image) based on the inputted image pickup signal. Furthermore, based on the control by the main control section 43, the optical system control section 42 causes the arm driving section 22g to operate such that the mobile optical system 22b is arranged at a position corresponding to the unmagnified observation magnification.

The user turns on the power sources of the respective sections of the endoscope apparatus 1, and thereafter moves the distal end portion 21a close to the object 101 in the body cavity of the test subject, while viewing the image displayed on the display device 5.

Furthermore, in order to locally observe the object 101, the user performs operation to bring the distal end portion 21a closer to the surface of the object 101, while issuing a magnification changing instruction for gradually changing the observation magnification from low magnification side to high magnification side by the zoom switch 25 (for example, maintaining the state where the button for increasing the observation magnification of the zoom switch 25 remains depressed).

On the other hand, while the magnification changing instruction from the low magnification side to the high magnification side is being issued by the zoom switch 25, the main control section 43 detects that the observation magnification is gradually increased from the unmagnified state and continues the control with respect to the optical system control section 42. In response to such a control, the optical system control section 42 causes the arm driving section 22g to operate such that the mobile optical system 22b moves from the low magnification side to the high magnification side.

In the case where the user performs the operations described above when locally observing the object 101, it is presumed that a predetermined correlation is established between the observation magnification and the distance from the distal end portion 21a to the surface of the object 101. Specifically, when the observation magnification is low, the distance from the distal end portion 21a to the surface of the object 101 is presumed to be relatively large. Furthermore, when the observation magnification gradually changes from low magnification to high magnification, it is presumed that the distance from the distal end portion 21a to the surface of the object 101 gradually becomes smaller. In addition, when the observation magnification gradually changes from high magnification to low magnification, it is presumed that the distance from the distal end portion 21a to the surface of the object 101 gradually becomes larger. If such a correlation is assumed to be established, the same correlation is presumed to be established between a predetermined observation magnification corresponding to the observation magnification immediately before the narrow-band light observation of at least one of a microstructure and a capillary vessel pattern of a mucosa surface layer of the object 101 becomes possible and the distance from the distal end portion 21a to the surface of the object 101 at the time that the observation magnification has become the predetermined magnification.

When detecting that the observation magnification has become equal to or larger than the predetermined observation magnification by constantly monitoring the increase of the observation magnification according to the magnification changing instruction by the zoom switch 25, the main control section 43 assumes that the distance from the distal end portion 21a to the surface of the object 101 has become suitable for the narrow-band light observation, and controls the rotary filter driving section 35 and the image processing section 41 to operate in the narrow-band light observation mode.

Then, the rotary filter driving section 35 drives the motors 33, 34 based on the control by the main control section 43 such that the second filter group 32B is interposed on the optical path of the white light source 31. In addition, based on the control by the main control section 43, the image processing section 41 performs operation to generate a narrow-band light observation image (psuedo-color image) based on the inputted image pickup signal.

According to the above-described working of the endoscope apparatus 1, the user can rapidly adjust the focus, while viewing the narrow-band light observation image of the object 101 displayed on the display device 5.

Note that, when the main control section 43 detects whether or not the observation magnification has become equal to or larger than the above-described predetermined observation magnification due to the increase of the observation magnification, the detection is not limited to be performed based on the monitoring result of the magnification changing instruction by the zoom switch 25, but may be performed based on the arrangement position of the mobile optical system 22b calculated based on the monitoring result of the control amount of the optical system control section 42, for example.

In addition, as far as the observation magnification is the observation magnification immediately before the narrow-band light observation of at least one of the microstructure and the capillary vessel pattern of the mucosa surface layer of the object 101 becomes possible, the above-described predetermined observation magnification may be appropriately set to a desired observation magnification for each user on an input screen or the like displayed on the display device. Alternatively, the predetermined observation magnification may be individually set for each endoscope, or may be a preset fixed observation magnification.

On the other hand, when completing the local observation of the object 101, the user performs operation to bring the distal end portion 21a away from the surface of the object 101, while issuing the magnification changing instruction for gradually changing the observation magnification from the high magnification side to the low magnification side by the zoom switch 25 (for example, maintaining the state where the button for decreasing the observation magnification of the zoom switch 25 remains depressed).

While the magnification changing instruction from the high magnification side to the low magnification side is being issued by the zoom switch 25, the main control section 43 detects that the observation magnification is gradually decreased from the observation magnification which is equal to or larger than the above-described predetermined observation magnification and continues the control with respect to the optical system control section 42. In response to such a control, the optical system control section 42 causes the arm driving section 22g to operate such that the mobile optical system 22b moves from the high magnification side to the low magnification side.

When detecting that the observation magnification has become smaller than the above-described predetermined observation magnification by constantly monitoring the decrease of the observation magnification according to the magnification changing instruction by the zoom switch 25, the main control section 43 assumes that the distance from the distal end portion 21a to the surface of the object 101 has become the distance which is not suitable for the narrow-band light observation, and performs the control to operate the rotary filter driving section 35 and the image processing section 41 in the normal light observation mode.

Then, the rotary filter driving section 35 drives the motors 33, 34 based on the control by the main control section 43 such that the first filter group 32A is interposed on the optical path of the white light source 31. In addition, based on the control by the main control section 43, the image processing section 41 performs operation to generate a normal light observation image (full-color image) based on the inputted image pickup signal.

Note that, when the main control section 43 detects whether or not the observation magnification has become smaller than the above-described predetermined observation magnification due to the decrease of the magnification observation, the detection is not limited to be performed based on the monitoring result of the magnification changing instruction by the zoom switch 25, but may be performed based on the arrangement position of the mobile optical system 22b calculated based on the monitoring result of the control amount of the optical system control section 42, for example.

As described above, according to the endoscope apparatus 1 of the present embodiment, based on the monitoring result of the increase of the observation magnification according to the magnification changing instruction, when it is detected that the observation magnification has become equal to or larger than the observation magnification immediately before the narrow-band light observation of at least one of the microstructure and the capillary vessel pattern of the mucosa surface layer of the object 101 becomes possible, the observation mode is switched from the normal light observation mode to the narrow-band light observation mode. That is, the endoscope apparatus 1 according to the present embodiment has the configuration and working in which the observation mode is switched from the normal light observation mode to the narrow-band light observation mode immediately before the timing that the focus adjustment is required in the operation actually performed by the user in the endoscopic observation of the local region of the object which exists in the living body. As a result, according to the endoscope apparatus 1 of the present embodiment, it is possible to reduce the time required for focus adjustment in performing endoscopic observation of the local region of the object which exists in the living body.

Note that the present invention is not limited to the embodiment described above, and various changes and modifications can be made without departing from the gist of the present invention.

The present application is filed claiming the priority of Japanese Patent Application No. 2009-095040 filed in Japan on April 9, 2009, and the above described disclosure is incorporated by reference in the present description, claims and drawings.

## Claims

1. An endoscope apparatus comprising:
an endoscope including an objective optical system capable of changing an observation magnification;
a light source device capable of selectively emitting broadband light including a visible light region and narrow-band lights of a plurality of wavelength bands obtained by discretizing light in the visible light region;
an observation magnification changing instruction section for issuing a magnification changing instruction for gradually increasing or decreasing the observation magnification of the objective optical system; and
a mode switching section for switching the light emitted from the light source device from one light to another light when detecting that the observation magnification gradually changes to reach a predetermined observation magnification based on the magnification changing instruction.

2. The endoscope apparatus according to claim 1, wherein the mode switching section switches light emitted from the light source device from the broadband light to the narrow-band lights of a plurality of wavelength bands when detecting that the observation magnification gradually increases to reach the predetermined observation magnification based on the magnification changing instruction.

3. The endoscope apparatus according to claim 2, wherein the mode switching section switches the light emitted from the light source device from the narrow-band lights of the plurality of wavelength bands to the broadband light, when detecting that the observation magnification gradually decreases to reach the predetermined observation magnification based on the magnification changing instruction.

4. The endoscope apparatus according to claim 1 or 2, wherein the predetermined observation magnification is an observation magnification immediately before observation of at least one of a microstructure and a capillary vessel pattern of a
mucosa surface layer with the narrow-band lights of the plurality of wavelength bands becomes possible.

5. The endoscope apparatus according to claim 4, wherein the predetermined observation magnification can be set to a desired observation magnification for each user.

6. The endoscope apparatus according to claim 4, wherein the predetermined observation magnification is set individually for each endoscope.
